Europäisches Patentamt

European Patent Office

Office européen des brevets

(11) Publication number: **0 339 814 B1**

(12) **EUROPEAN PATENT SPECIFICATION**

(45) Date of publication of patent specification: **20.10.93**

(51) Int. Cl.5: **G01N 33/573**, G01N 33/70, C12Q 1/48, G01N 33/577, C12P 21/08

(21) Application number: **89303413.2**

(22) Date of filing: **06.04.89**

(54) **Reagent for measuring creatine kinase activity and measuring method thereof.**

(30) Priority: **06.04.88 JP 86163/88**

(43) Date of publication of application:
**02.11.89 Bulletin  89/44**

(45) Publication of the grant of the patent:
**20.10.93 Bulletin  93/42**

(84) Designated Contracting States:
**DE FR GB IT**

(56) References cited:
**EP-A- 0 178 113**
**EP-A- 0 227 440**

**CHEMICAL ABSTRACTS, vol. 109, 1988, Columbus, OH (US); p. 508, no. 36622n&NUM;**

**CHEMICAL ABSTRACTS, vol. 109, 1988, Columbus, OH (US); p. 499, no. 5160f&NUM;**

**BIOLOGICAL ABSTRACTS, vol. 33, no. 6, 1987, Philadelphia, PA (US); J.L.DAISS, p. 986, no. RRM/33073450&NUM;**

(73) Proprietor: **UNITIKA LTD.**
**50, Higashi Hommachi 1-chome**
**Amagasaki-shi Hyogo-ken(JP)**

Proprietor: **IATRON LABORATORIES INC.**
**11-4 Higashi-Kanda 1-chome**
**Chiyoda-ku Tokyo 101(JP)**

(72) Inventor: **Suzuki, Tadao**
**151-30-3D-1109, Mukaijimaninomaru-cho**
**Fushimi-ku Kyoto-shi(JP)**
Inventor: **Kamei, Tomoko**
**21-3-A2-304, Daigouenoyama-cho**
**Fushimi-ku Kyoto-shi Kyoto-fu(JP)**
Inventor: **Era, Mihoko**
**21 Koaza Ayaugigahara**
**Oaza Hirao**
**Yamashiro-cho Soraku-gun Kyoto-fu(JP)**
Inventor: **Tsubota, Hiroyuki**
**2-11-12-304 Imai**
**Chiba-shi Chiba-ken(JP)**

(74) Representative: **Atkinson, Peter Birch et al**
**MARKS & CLERK**
**Suite 301**
**Sunlight House**
**Quay Street**
**Manchester M3 3JY (GB)**

**Description**

FIELD OF THE INVENTION

The present invention relates to a reagent for measuring creatine kinase activity of body fluids and a measuring method thereof.

BACKGROUND OF THE INVENTION

Measurement of creatine kinase (CK) activity of body fluids, such as serum is very significant for a diagnosis of muscle diseases, especially myocardial infarction. Creatine kinase (CK) has three isozymes based on a combination of two subunits, such as creatine kinase (CK) MM, creatine kinase (CK) MB and creatine kinase (CK) BB. Creatine kinase (CK) MM is mainly present in muscles, such as skeletal muscles, cardiac muscles and the like, creatine kinase (CK) MB is present in cardiac muscles and creatine kinase (CK) BB is in internal organs, such as a brain, the kidney and the like. Creatine kinase (CK) MB which is specifically present in cardiac muscles is important as a marker for a diagnosis of cardiac infarction.

Accordingly, reagents which specifically measure creatine kinase (CK) MB are proposed. Japanese Patent Publications (examined) 19239/1981 and 20274/1983 disclose a method wherein an antibody, which specifically inhibits M subunits in creatine kinase (CK) MM and MB, is added and activity of the residual B subunit is then measured. The present inventors proposed to improve the above method by using a monoclonal antibody as an inhibiting antibody as disclosed in EP-A-0 261 781 (corresponding to Japanese Patent Application Ser. No. 191899/1986). These methods are very effective, but it takes more than 10 hours for CK-MB to excess normal range (see Clin., Chem. by Wu, 33, p.358 (1987)). It is difficult to diagnose myocardial infarction early by measurement of CK-MB. It therefore is required to develop a new diagnostic method which enables early diagnosis of myocardial infarction.

It has been recently elucidated that M subunits in creatine kinase (CK) MM and MB which is released from the heart or skeletal muscles due to myocardial infarction or a muscles disease is modified by carboxypeptitase N in blood plasma to M' subunit (modified M subunit) (J. Biol. Chem. by George et al., 259, p 2667 (1984). Thus, creatine kinase (CK) MM is converted from $MM_3$ (tissue type MM) through $MM_2$ - (hybrid type MM') to $MM_1$ (serum type M'M'). It is also made clear in Circulation by Hashimoto et al., 71 p 366 (1985) that in normal condition, a ratio of $MM_3/MM_1$ is approximately 0.3, but, in myocardial infarction, the ratio shows about 2 after 3 to 6 hours from after onset of myocardial infarction. Accordingly, the measurement of the ratio of $MM_3/MM_1$ makes possible an early diagnosis of myocardial infarction. Such early diagnosis of myocardial infarction is significant, because it is essential how early a intercoronary thrombolytic therapy is applied to a patient. Reattack of infarction is also easily and early detected.

The measurement of the ratio of $MM_3/MM_1$ is presently carried out by an activity measuring method after separation by electrophoresis, a chromatofocusing method, an isoelctric point electrophoresis and the like. These methods are very complicated and time consuming. As mentioned above, since it is important that the measurement of the ratio of $MM_3/MM_1$ is made fast, these methods are not good. It is desired to develop a method which can measure the ratio in a short time.

SUMMARY OF THE INVENTION

The present invention provides such a measuring method of the ratio ($MM_3/MM_1$) and a reagent therefor. Accordingly, the present invention provides a reagent for measuring creatine kinase (CK) activity comprising an antibody, wherein said antibody inhibits creatine kinase (CK) M subunit, but does not inhibit creatine kinase (CK) M' subunit.

The present invention also provides a method for measuring creatine kinase (CK) activity comprising determining an inhibition of creatine kinase (CK) of body fluids using a reagent for measuring creatine kinase (CK) activity, wherein said reagent comprises an antibody which inhibits creatine kinase (CK) M subunit, but does not inhibit creatine kinase (CK) M' subunit.

DETAILED DESCRIPTION OF THE INVENTION

The antibody of the present invention can be either polyclonal or monoclonal, but a monoclonal antibody is preferred. Physicochemical characteristics of the antibody are shown as follow;

3

(1) Action

The antibody acts on creatine kinase (CK) to raise an antigen-antibody reaction, then inhibits creatine kinase (CK) M subunit activity.

(2) Reaction specificity

The antibody inhibits only M subunit of creatine kinase (CK) M subunit, but does not inhibit M′ subunit.

(3) Optimum pH

6 to 9

(4) Suitable pH range

3 to 11

(5) Measuring method of potency

Measured by a dilution rate which is detected from the measurement of inhibition.

(6) Suitable temperature range for action

0 to 40 °C

(7) Condition of inactivation

The antibody is inactivated at 100 °C for 30 minutes.

(8) Molecular weight

130,000 to 210,000
For obtaining the monoclonal antibody, a hybridoma cell strain which can produce a monoclonal antibody inhibiting creatine kinase (CK) M activity is necessary. Such a hybridoma cell strain has the following cytological characteristics;

(1) Derivation

Fused cells of lymphocytes producing anti-CK-M antibody and myeloma cells.

(2) Shape

The fused cells have similar shape to myeloma cells. They have a size of 10 to 20 micrometer.

(3) Function

They constantly produce monoclonal antibodies inhibiting CK-M activity which recognize one antigenic determinant.

(4) Growth ability

They show proliferation ability similar to myeloma cells. Thus, they proliferate about ten times in number for 72 hours.

(5) Storage stability

They are stored for a long period of time at a temperature of not more than -120 °C.

(6) Optimum proliferation conditions

37 °C, pH 7.2

(7) Proliferation range

32 to 42 °C, pH 6.5 to 7.8

For obtaining such cell strain, hybridomas which produce a monoclonal antibody inhibiting CK-M activity are obtained by, for example, a method described in EP-A-0 261 781 from which some hybridomas which do not inhibit M' subunit are selected in a similar manner mentioned above. The method disclosed in EP-A-0261 781 for producing the hybridoma cell line comprises fusing a sensitized lymphocyte obtained from a creatine kinase-immunised animal and a myeloma cell, said cell line being capable of producing a monoclonal antibody which is reactive with creatine kinase and which inhibit creatine kinase M subunit activity.

As mentioned above, hybridomas producing antibodies which inhibit CK-MM$_3$, but do not inhibit CK-MM$_1$ are obtained. One of hybridomas which is actually obtained by the above mentioned method is deposited on January 26,, 1988 at Fermentation Research Institute Agency of Industrial Science and Technology, Ibaraki-ken, Japan to assign a number of FERM P-9839. These hybridomas are named CKH-5 and can be permanently stored at -120° C.

The hybridomas are cultured and an antibody is obtained therefrom. The obtaining method of the antibody is known to the art, especially a method described in Japanese Patent Application Ser. No. 191899/1986. The obtained antibodies may be used intact. They may be treated with proteolytic enzymes, such as pepsin, papain and the like to form Fab, Fab', F(ab')$_2$ fragments.

The reagent for measuring creatine kinase activity is prepared and used as generally described in Japanese Patent Publications (examined) 19239/1981 and 20274/1983 and EP-A-0 261 781. For example, the reagent is generally composed of two packages. The first package generally contains 0.1 to 40 unit/ml of glucokinase (GlcK) or hexokinase (HK), 0.1 to 40 unit/ml of glucose-6 phosphate dehydrogenase (G6PDH), 0.001 to 5 mg/ml of M subunit inhibiting antibody (Ab), 0.1 to 20 mM of adenosine diphosphate (ADP), 0.05 to 20 mM of nicotinamide adenine dinucleotide (NAD$^+$) or nicotinamide adenine dinucleotide phosphate (NADP$^+$) 1 to 200 mM of glucose, 0.2 to 20 mM of adenosine monophosphate (AMP), 0.001 to 0.1 mM of diadenosine pentophosphate (Ap5A), 0.5 to 50 mM of N-acetylcysteine (NAC) or another thiol compound, 0.5 to 30 mM of magnesium salts (e.g. magnesium acetate), 0.5 to 50 mM of sodium azide, 0.1 to 20 mM of ethylenediamine tetraacetate (EDTA) and 5 to 50 mM of a buffer solution (pH 6.7). The second reagent contains 2 to 70 mM of creatine phosphate (CrP), 0.5 to 50 mM of sodium azide and 5 to 500 mM of a buffer solution. Preferably, in the first reagent, GlcK or HK is present in an amount of 0.2 to 20 unit/ml, G6PDH is in an amount of 0.2 to 20 unit/ml, Ab is 0.005 to 2 mg/ml, ADP is 0.2 to 10 mM, NAD$^+$ or NADP$^+$ is 0.1 to 10 mM, glucose is 2 to 100 mM, AMP is 0.5 to 15 mM, Ap5A is 0.002 to 0.050 mM, NAC is 2 to 30 mM, magnesium salts are 1 to 15 mM, sodium azide is 1 to 30 mM, EDTA is 0.2 to 10 mM and the buffer solution (pH 6.7) is 10 250 mM. In the second reagent, preferably CrP is present in an mount of 5 to 40 mM, sodium azide is in an amount of 1 to 30 mM and the buffer solution 10 to 250 mM.

As a measuring method, 0.5 ml of the first reagent is mixed with 0.001 to 0.02 ml of an enzyme solution (or serum) containing creatine kinase and kept at 25 to 37 °C for 5 to 10 minutes. Then, 0.125 ml of the second reagent is added to it and the residual creatine kinase activity is measured by an absorption change of 340 nm using a spectrometer. Thereafter, as a control, the first reagent which does not contain the antibody is added to the enzyme solution (or serum) and measured in a same manner as mentioned above. An inhibition rate is given by the following formula from the residual creatine kinase (CK) activity and the control creatine kinase (CK) activity;

$$\text{Inhibition rate (\%)} = \frac{\text{Control activity} - \text{Residual activity}}{\text{Control activity}} \times 100$$

The antibody of the present invention does not inhibit creatine kinase (CK)-M' subunit, but inhibits creatine kinase (CK)-M subunit. M subunit of creatine kinase which has been released from the heart by myocardial infarction is modified with carboxypeptidase of blood. Thus, CK-MM(MM$_3$) is modified through MM'(MM$_2$) to M'M'(MM$_1$), and CK-MB is modified to M'B(MB$_1$) with the passage of time. A large amount of

5

creatine kinase just after released from the heart is modified very little so as to obtain a high inhibition. As proceeding the modification, the inhibition is lowered. Accordingly, by measuring an inhibition with the reagent of the present invention, myocardial infarction is early examined and a time when a patient fall in disease is estimated.

EXAMPLES

The present invention will be illustrated by the following Examples.

Reference Example 1

An emulsion mixture of 50 microgram of pig CK-MM with complete Freund adjuvant (available from Nakarai Chemical Company) in a ratio of 1:1 was administered to 8 weeks mouse Balb/c (available from Nippon Clea Company). After three weeks, 50 microgram of pig CK-MM was intravenously injected. It was then fused with mouse myeloma X63.6.5.3 by a method by Oi, V.T., Selected Methods in Cellular Immunology, p. 351, W.H. Freeman and Co., 1980 and hybridoma grew in 54 wells / 96 wells. The supernatant was added to a reagent for measuring creatine kinase (CK) activity of the following Reference Example 2 and an inhibition of CK-MM was measured with respect to CK-MM$_3$ and CK-MM$_1$ to select antibodies which does not inhibit CK-MM$_1$ but inhibits CK-MM$_3$. The selected strain was cloned by a limit dilution method to obtain a hybridoma CKH-5 of monoclone. The CKH-5 shows the same cytological properties as mentioned before.

The hybridoma CKH-5 was cultured on RPM11640 culture medium containing 10 % fetal bovine serum to a cell number of $2 \times 10^6$ /ml, 300 ml of which was then subjected to centrifugal separation to collect the supernatant. It was then fractionated with ammonium sulfate to obtain crude antibody fraction, and absorbed to Protein A Cephalose (pH 8.0) after dialyzing. The absorbed fraction was eluted with a citric acid buffer solution to obtain a monoclonal antibody (CKA-5) of 3.2 mg.

Reference Example 2

The antibody was added to reagents for measuring creatine kinase (CK) activity to determine inhibition of CK-MM$_3$, MM$_2$ and MM$_1$ in serum separated by a chromatofocusing.

The first reagent was prepared by mixing 1.4 unit/ml of GlcK derived from Bacillus stearothermophilus - (available from Seikagaku Kogyo Co., Ltd.), 1.2 unit/ml of G6PDH derived from Leuconostoc mesenteroides (available from Oriental Yeast Co., Ltd.), 1.2 mM of ADP, 0.75 mM of NADP, 25 mM of glucose, 6.25 mM of AMP, 125 micro M of Ap5A, 12.5 mM of N-acetylcystein, 12.5 mM of magnesium acetate, 10 mM of sodium azide, 2.5 mM of EDTA and 150 mM of an imidazole-acetate buffer solution (pH 6.7). The second reagent was prepared by mixing 100 mM of CP, 10 mM of sodium azide and 25 mM of a Tris-acetate buffer solution (pH 8.5).

The monoclonal antibody CKA-5 inhibiting CK-M activity was added to the first reagent and CK-MM$_3$, then MM$_2$ or MM$_1$ was added to 0.5 ml of the mixture. It was then charged in a cell having a light length of 1 cm and incubated for 15 minutes. Next, 0.125 ml of the second reagent was added and the residual CK activity was measured by an absorption of 340 nm using a spectrometer which was kept at 30 °C. As control, CK-MM$_3$, MM$_2$ or MM$_1$ was added to the reagent which does not contain the antibody, and subjected to a same measuring. The result is shown in Table 1.

Table 1

| Inhibition activity of CKA-5 | | | |
|---|---|---|---|
| Isoform | Activity with no antibody (u/l) | Activity with antibody (u/l) | Inhibition (%) |
| CK-MM$_3$ | 221 | 1 | 100 |
| CK-MM$_2$ | 210 | 101 | 52 |
| CK-MM$_1$ | 197 | 195 | 1 |
| Note: the values are an average of three samples. | | | |

As shown in Table 1, CKA-5 does not inhibit CK-MM$_1$ of serum type, but inhibits CK-MM$_3$ of tissue type. It also inhibits a half of MM$_2$ of hybrid type. Thus, CKA-5 does not inhibit M′ subunit, but inhibits M subunit, and is inside of the physicochemical characteristics.

Reference Example 3 and Comparative Example 1

CK-MM (800U/l) purified from cardiac muscles was added to blood plasma and incubated at 37 °C. Inhibition of CK-MM by CKA-5 was determined with time as generally described in Reference Example 2. A diagnostic reagent for measurement of CK-MB (CK-MB kit) which contains CK-MM inhibiting antibody (Boehringer Mannheim Company) was employed as a comparative example and inhibition was measured. The result is shown in Table 2.

Table 2

| Change with time of inhibition by CKA-5 | | |
|---|---|---|
| Incubation time (hours) | Inhibition by CKA-5 (%) | Inhibition by CK-MB kit (%) |
| 0 | 97.3 | 100 |
| 1 | 57.6 | 100 |
| 2 | 38.9 | 100 |
| 3 | 23.5 | 100 |
| 4 | 16.1 | 97.0 |
| 5 | 8.2 | 96.7 |
| 6 | 0 | 100 |

As the purified CK-MM(MM$_3$) is modified by carboxy peptitase N in blood plasma to MM$_2$ and MM$_1$, inhibition of CK-MM by CKA-5 reduces with time and 6 hours later there is no inhibition. This is corresponded to the change of isoform. On the other hand, the inhibition of CK-MM by CK-MB kit does not change after 6 hours and therefore this can not trace the change of isoform.

Example 1 and Comparative Example 2

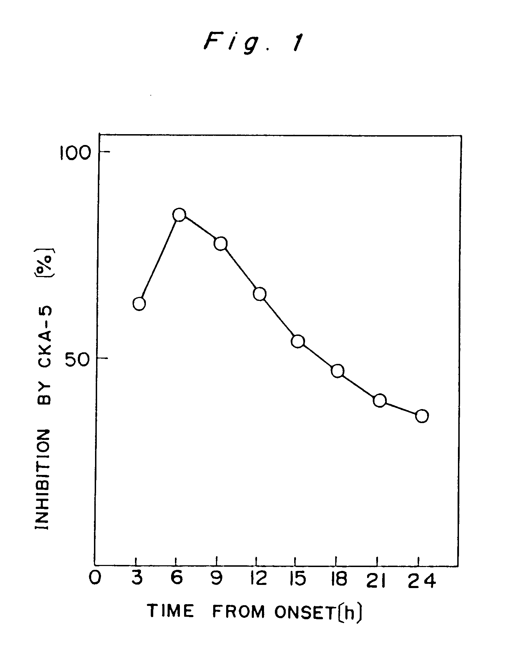

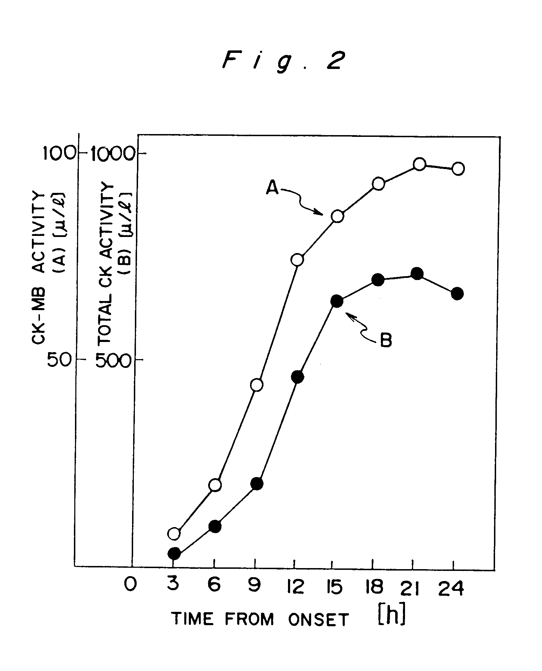

As generally described in Reference Example 2, the CK activity and inhibition by CKA-5 of the serum from patient A who had onset of myocardial infarction was determined with time. For a comparison, the activity of CK-MB was determined using a reagent for CK-MB measurement kit which contains a CK-MM inhibiting antibody. The result is shown in Fig.1 and Fig.2. As shown in the figures, the inhibition by CKA-5 shows a peak after 6 hours from the onset, but CK-MB which has been used for a diagnosis of myocardial infarction shows a peak after 21 hours from the onset. Accordingly, it is very clear that the measurement of the inhibition by CKA-5 is very useful for an early diagnosis of myocardial infarction.

Example 2 and Comparative Example 3

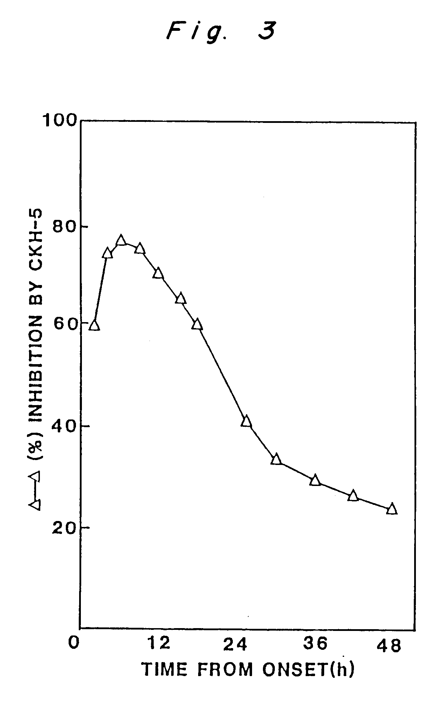

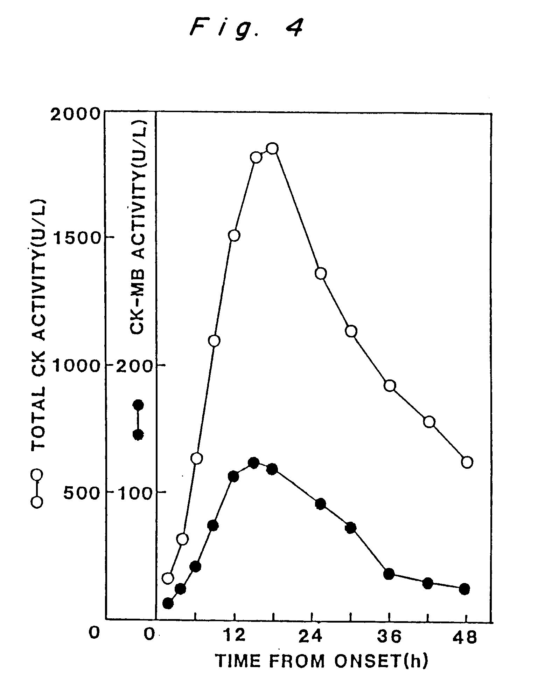

As generally described in Example 1, the CK activity and inhibition by CKA-5 of the serum from patient B who had a fit of myocardial infarction was determined with time. For a comparison, the activity of CK-MB was determined as generally described in Comparative Example 2. The result is shown in Fig.3 and Fig.4. As shown in the figures, the inhibition by CKA-5 shows a peak after 6 hours from the onset, but that of CK-MB which has been used for a diagnosis of myocardial infarction shows a peak after 16 hours from the onset. Accordingly, it is very clear that the measurement of the inhibition by CKA-5 is very useful for an early diagnosis of myocardial infarction.

## BRIEF EXPLANATION OF THE DRAWINGS

Figs.1 and 3 show a relation of an inhibition by CKA-5 with time after onset of myocardial infarction.

Figs.2 and 4 show a relation of a total CK activity (A) and CK-MB activity (B) with time after onset of myocardial infarction.

## Claims

1. A reagent for measuring creatine kinase activity comprising an antibody, wherein said antibody inhibits creatine kinase M subunit, but does not inhibit creatine kinase M′ subunit.

2. The reagent according to Claim 1 wherein said antibody is obtained from a hybridoma cell strain which can produce a monoclonal antibody inhibiting creatine kinase (CK) M activity.

3. The reagent according to Claim 1 being composed of two packages, one of which comprises 0.1 to 40 unit/ml of glucokinase (GlcK) or hexokinase (HK), 0.1 to 40 unit/ml of glucose-6 phosphate dehydrogenase (G6PDH), 0.001 to 5 mg/ml of M subunit inhibiting antibody (Ab), 0.1 to 20 mM of adenosine diphosphate (ADP), 0.05 to 20 mM of nicotinamide adenine dinucleotide ($NAD^+$) or nicotinamide adenine dinucleotide phosphate ($NADP^+$), 1 to 200 mM of glucose, 0.2 to 20 mM of adenosine monophosphate (AMP), 0.001 to 0.1 mM of diadenosine pentophosphate (Ap5A), 0.5 to 50 mM of N-acetylcysteine (NAC) or another thiol compound, 0.5 to 30 mM of magnesium salts, 0.5 to 50 mM of sodium azide, 0.1 to 20 mM of ethylenediamine tetraacetate (EDTA) and 5 to 50 mM of a buffer solution (pH 6.7), and the other reagent comprises 2 to 70 mM of creatine phosphate (CrP), 0.5 to 50 mM of sodium azide and 5 to 500 mM of a buffer solution.

4. A method for measuring creatine kinase activity comprising determining an inhibition of creatine kinase of body fluids using a reagent for measuring creatine kinase activity, wherein said reagent comprises an antibody which inhibits creatine kinase M subunit, but does not inhibit creatine kinase M′ subunit.

5. The method according to Claim 4 wherein said antibody is obtained from a hybridoma cell strain which can produce a monoclonal antibody inhibiting creatine kinase (CK) M activity.

6. The method according to Claim 4 wherein said reagent is composed of two packages, one of which comprises 0.1 to 40 unit/ml of glucokinase (GlcK) or hexokinase (HK), 0.1 to 40 unit/ml of glucose-6 phosphate dehydrogenase (G6PDH), 0.001 to 5 mg/ml of M subunit inhibiting antibody (Ab), 0.1 to 20 mM of adenosine diphosphate (ADP), 0.05 to 20 mM of nicotinamide adenine dinucleotide ($NAD^+$) or nicotinamide adenine dinucleotide phosphate ($NADP^+$), 1 to 200 mM of glucose, 0.2 to 20 mM of adenosine monophosphate (AMP), 0.001 to 0.1 mM of diadenosine pentophosphate (Ap5A), 0.5 to 50 mM of N-acetylcysteine (NAC) or another thiol compound, 0.5 to 30 mM of magnesium salts, 0.5 to 50 mM of sodium azide, 0.1 to 20 mM of ethylenediamine tetraacetate (EDTA) and 5 to 50 mM of a buffer solution (pH 6.7), and the other reagent comprises 2 to 70 mM of creatine phosphate (CrP), 0.5 to 50 mM of sodium azide and 5 to 500 mM of a buffer solution.

## Patentansprüche

1. Reagens zur Messung der Creatinkinase-Aktivität, umfassend einen Antikörper, wobei der Antikörper die M-Untereinheit der Creatinkinase hemmt, aber nicht die M'-Untereinheit der Creatinkinase hemmt.

2. Reagens nach Anspruch 1, dadurch **gekennzeichnet**, daß der Antikörper aus einem Hybridoma-Zellstamm erhalten worden ist, der einen monoclonalen Antikörper erzeugen kann, der die Creatinkinase (CK) M-Aktivität hemmt.

3. Reagens nach Anspruch 1, dadurch **gekennzeichnet**, daß es zwei Packungen enthält, von denen eine 0,1 bis 40 Einheiten/ml Glucokinase (GlcK) oder Hexokinase (HK), 0,1 bis 40 Einheiten/ml Glucose-6-phosphat-Dehydrogenase (G6PDH), 0,001 bis 5 mg/ml des, die M-Untereinheit hemmenden Antikörpers (Ab), 0,1 bis 20 mM Adenosin-Diphosphat (ADP), 0,05 bis 20 mM Nicotinamid-Adenin-Dinucleotid ($NAD^+$) oder Nicotinamid-Adenin-Dinucleotid-Phosphat ($NADP^+$), 1 bis 200 mM Glucose, 0,2 bis 20 mM Adenosin-Monophosphat (AMP), 0,001 bis 0,1 mM Diadenosin-Pentaphosphat (Ap5A), 0,5 bis 50 mM N-Acetylcystein (NAC) oder eine andere Thiolverbindung, 0,5 bis 30 mM Magnesiumsalze, 0,5 bis 50 mM Natriumazid, 0,1 bis 20 mM Ethylendiamintetraacetat (EDTA) und 5 bis 50 mM einer Pufferlösung (pH 6,7) enthält, und das andere Reagens 2 bis 70 mM Creatinphosphat (CrP), 0,5 bis 50 mM Natriumazid und 5 bis 500 mM einer Pufferlösung enthält.

4. Verfahren zur Messung der Creatinkinase-Aktivität, dadurch **gekennzeichnet**, daß man eine Hemmung der Creatinkinase in Körperflüssigkeiten unter Verwendung eines Reagenses zur Messung der Creatinkinase-Aktivität bestimmt, wobei das Reagens einen Antikörper, der die M-Untereinheit der Creatinkinase hemmt, aber die M'-Untereinheit der Creatinkinase nicht hemmt, umfaßt.

5. Verfahren nach Anspruch 4, dadurch **gekennzeichnet**, daß der Antikörper aus einem Hybridoma-Zellstamm erhalten wurde, der einen monoclonalen Antikörper, der die Creatinkinase (CK) M-Aktivität hemmt, erzeugen kann.

6. Verfahren nach Anspruch 4, dadurch **gekennzeichnet**, daß das Reagens zwei Packungen enthält, wobei eine 0,1 bis 40 Einheiten/ml Glucokinase (GlcK) oder Hexokinase (HK), 0,1 bis 40 Einheiten/ml Glucose-6-phosphat-Dehydrogenase (G6PDH), 0,001 bis 5 mg/ml des, die M-Untereinheit hemmenden Antikörpers (Ab), 0,1 bis 20 mM Adenosin-Diphosphat (ADP), 0,05 bis 20 mM Nicotinamid-Adenin-Dinucleotid (NAD$^+$) oder Nicotinamid-Adenin-Dinucleotid-Phosphat (NADP$^+$), 1 bis 200 mM Glucose, 0,2 bis 20 mM Adenosin-Monophosphat (AMP), 0,001 bis 0,1 mM Diadenosin-Pentaphosphat (Ap5A), 0,5 bis 50 mM N-Acetylcystein (NAC) oder eine andere Thiolverbindung, 0,5 bis 30 mM Magnesiumsalze, 0,5 bis 50 mM Natriumazid, 0,1 bis 20 mM Ethylendiamintetraacetat (EDTA) und 5 bis 50 mM einer Pufferlösung (pH 6,7) enthält, und das andere Reagens 2 bis 70 mM Creatinphosphat (CrP), 0,5 bis 50 mM Natriumazid und 5 bis 500 mM einer Pufferlösung enthält.

## Revendications

1. Réactif pour mesurer l'activité de créatine kinase, comprenant un anticorps, dans lequel ledit anticorps inhibe la sous-unité M de la créatine kinase, mais n'inhibe pas la sous unité M' de la créatine kinase.

2. Réactif selon la revendication 1, dans lequel ledit anticorps est obtenu à partir d'une lignée cellulaire d'hybridomes qui peut produire un anticorps monoclonal inhibant l'activité de créatine kinase (CK) M.

3. Réactif selon la revendication 1, composé de deux parties dont l'une comprend 0,1 à 40 unités/ml de glucokinase (GlcK) ou d'hexokinase (HK), 0,1 à 40 unités/ml de glucose-6 phosphate déshydrogénase (G6PDH), 0,001 à 5 mg/ml d'anticorps (Ab) inhibant la sous-unité M, 0,1 à 20 mM d'adénosine diphosphate (ADP), 0,05 à 20 mM de nicotinamide adénine dinucléotide (NAD$^+$), ou de nicotinamide adénine dinucléotide phosphate (NADP$^+$), 1 à 200 mM de glucose, 0,2 à 20 mM d'adénosine monophosphate (AMP), 0,001 à 0,1 mM de diadénosine pentophosphate (Ap5A), 0,5 à 50 mM de N-acétylcystéine (NAC) ou d'un autre composé thiol, 0,5 à 30 mM de sels de magnésium, 0,5 à 50 mM d'azoture de sodium, 0,1 à 20 mM d'éthylènediamine tétracétate (EDTA) et 5 à 50 mM d'une solution tampon (pH 6,7), et l'autre partie comprend 2 à 70 mM de créatine phosphate (CrP), 0,5 à 50 mM d'azoture de sodium et 5 à 500 mM d'une solution tampon.

4. Procédé pour mesurer l'activité de créatine kinase comprenant la détermination d'une inhibition de la créatine kinase de fluides corporels en utilisant un réactif pour mesurer l'activité de créatine kinase, dans lequel ledit réactif comprend un anticorps qui inhibe la sous-unité M de la créatine kinase, mais n'inhibe pas la sous-unité M'de la créatine kinase.

5. Procédé selon la revendication 4, dans lequel ledit anticorps est obtenu à partir d'une lignée cellulaire d'hybridomes qui peut produire un anticorps monoclonal inhibant l'activité de créatine kinase (CK) M.

6. Procédé selon la revendication 4, dans lequel ledit réactif est composé de deux parties dont l'une comprend 0,1 à 40 unités/ml de glucokinase (GlcK) ou d'hexokinase (HK), 0,1 à 40 unités/ml de glucose-6 phosphate déshydrogénase (G6PDH), 0,001 à 5 mg/ml d'anticorps (Ab) inhibant la sousunité M, 0,1 à 20 mM d'adénosine diphosphate (ADP), 0,05 à 20 mM de nicotinamide adénine dinucléotide (NAD$^+$), ou de nicotinamide adénine dinucléotide phosphate (NADP$^+$), 1 à 200 mM de glucose, 0,2 à 20 mM d'adénosine monophosphate (AMP), 0,001 à 0,1 mM de diadénosine pentophosphate (Ap5A),

9

0,5 à 50 mM de N-acétylcystéine (NAC) ou d'un autre composé thiol, 0,5 à 30 mM de sels de magnésium, 0,5 à 50 mM d'azoture de sodium, 0,1 à 20 mM d'éthylènediaminetétracétate (EDTA) et 5 à 50 mM d'une solution tampon (pH 6,7), et l'autre partie comprend 2 à 70 mM de créatine phosphate (CrP), 0,5 à 50 mM d'azoture de sodium et 5 à 500 mM d'une solution tampon.

Fig. 1

# Fig. 2

EP 0 339 814 B1

# Fig. 3

Fig. 4